(19) Europäisches Patentamt
European Patent Office
Office européen des brevets

(11) Veröffentlichungsnummer : **0 262 201 B1**

# (12) EUROPÄISCHE PATENTSCHRIFT

(45) Veröffentlichungstag der Patentschrift :
**27.02.91 Patentblatt 91/09**

(51) Int. Cl.[5] : **C07J 1/00,** C07J 21/00, C07J 51/00, C07J 53/00

(21) Anmeldenummer : **87902382.8**

(22) Anmeldetag : **09.04.87**

(86) Internationale Anmeldenummer :
**PCT/DE87/00156**

(87) Internationale Veröffentlichungsnummer :
**WO 87/06237 22.10.87 Gazette 87/23**

## (54) VERFAHREN ZUR HERSTELLUNG VON 7-g(a)-PROPYLSTEROIDEN.

(30) Priorität : **11.04.86 DE 3612632**

(43) Veröffentlichungstag der Anmeldung :
**06.04.88 Patentblatt 88/14**

(45) Bekanntmachung des Hinweises auf die Patenterteilung :
**27.02.91 Patentblatt 91/09**

(84) Benannte Vertragsstaaten :
**AT BE CH DE FR GB IT LI LU NL SE**

(56) Entgegenhaltungen :
**EP-A- 0 018 245**
**DE-A- 3 234 972**

(56) Entgegenhaltungen :
**Journal of the American ChemicalSociety, Band 105, Nr. 8, 20.April 1983, American Chemical Society, (Washington, DC,US), T.A.Blumenkopf et al.: "Stereochemistry of the Sakurai reaction. Additions to cyclohexenones and cycloheptenones", siehe Seiten 2354-2358, in der Anmeldung erwähnt**

(73) Patentinhaber : **SCHERING AKTIENGESELLSCHAFT**
**Müllerstrasse 170/178 Postfach 65 03 11**
**D-1000 Berlin 65 (DE)**

(72) Erfinder : **NICKISCH, Klaus**
**Alt Lichtenrade 11**
**D-1000 Berlin 49 (DE)**
Erfinder : **LAURENT, Henry**
**Glambecker Weg 21**
**D-1000 Berlin 28 (DE)**

**EP 0 262 201 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 7α-Propylsteroiden.

7α-Propylsteroide sind in letzer Zeit durch ihr pharmakologisches Wirkungsspektrum hervorgetreten. In Steroids 40, 603 (1982) wird z.B. die Aromatasehemmende Wirkung des 7α-Propyl-4-androsten-3,17-dion beschrieben. Aus dem US-Patent 4,258,039, aus der deutschen Offenlegungsschrift 3 234 972 und aus Eur.J.Clin.Pharmacol. 28, 531(1985) sind die Aldosteronantagonistisch wirksamen 7α-Propyl-17-spirolactone sowie auch deren ringgeöffnete Kaliumsalze bekannt. 7-Alkylsteroide lassen sich entweder durch Kupferchlorid-katalysierte Addition von Alkylmagnesiumhalogeniden [J.Am.Chem.Soc. 81, 4069(1959)] oder durch Anlagerung von Kupfer-Lithium-alkylverbindungen [Steroids 27, 759(1976)] an 4,6-ungesättigte 3-Ketosteroide herstellen. Dabei entstehen je nach Struktur der Ausgangsverbindungen Gemische mit unterschiedlichen Anteilen an 7α- und 7β-alkylierten Verbindungen. Das pharmakologisch interessantere 7α-Isomere kann nur durch aufwendige chromatographische Trennverfahren isoliert werden. Es zeigte sich nun überraschend, daß man eine Propenylgruppe stereoselektiv in die 7α-Position einführen kann, wenn man 4,6-ungesättigte 3-Ketosteroide mit Allyltrialkylsilanen oder Allyltrialkylstannanen in Gegenwart von Lewissäuren umsetzt. In der nichtsteroidalen Chemie wurde die Anlagerung von Allyltrimethylsilan an ungesättigte Carbonylverbindungen bereits durchgeführt. Dabei sind allerdings stets Gemische verschiedener Stereoisomere erhalten worden [vgl. J.Am.Chem.Soc. 105, 2354(1983) und Tetrahedron Letters 1979, 4557].

Die Erfindung betrifft somit ein Verfahren zur Herstellung von 7α-Propylsteroiden der allgemeinen Formel I,

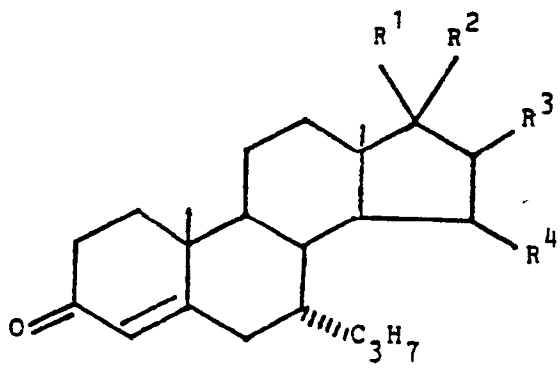

(I) ,

worin

$R^1$ eine β-Hydroxygruppe und

$R^2$ Wasserstoff oder den α-gebundenen Rest $-CH_2-CH_2COOR^5$,

$R^1$ und $R^2$ gemeinsam den Rest

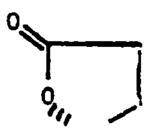

,

$R^3$, $R^4$ Wasserstoff,

$R^3$ und $R^4$ gemeinsam einen Methylenrest, der α- oder β-gebunden sein kann, und

$R^5$ Wasserstoff, Natrium oder Kalium bedeuten kann, dadurch gekennzeichnet, daß man 4,6-ungesättigte Steroidketone der allgemeinen Formel II

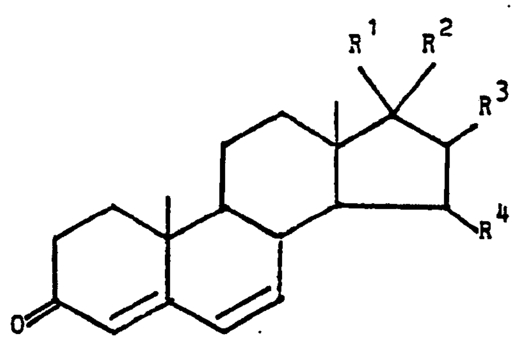

(II),

worin $R^1$-$R^4$ die oben genannten Bedeutungen haben, mit einer Allyltrialkylverbindung der allgemeinen Formel III,

$$CH_2{=}CH{-}CH_2{-}X(Alkyl)_3 \qquad (III)\ .$$

worin X die Bedeutung Si oder Sn und Alkyl die

Bedeutung $C_{1-4}$-Alkyl haben, in Gegenwart einer Lewissäure umsetzt und anschließend die gebildete 7α-Propenyl-Seitenkette selektiv hydriert.

Als Lewissäuren kommen $AlCl_3$, $SnCl_4$, $SnCl_2$, $BF_3$-Etherat, $TiCl_4$, $AlCl(C_2H_5)_2$ und $AlCl_2(C_2H_5)$ in Betracht. Bevorzugte Lewissäuren sind $TiCl_4$ und $AlCl(C_2H_5)_2$. Alkyl in Verbindungen der allgemeinen Formel III bedeutet Methyl, Ethyl, n-Propyl, Isopropyl, n-Butyl, Isobutyl.

Als Katalysator für die selektive Hydrierung der 7α-Propenylgruppe kann vorzugsweise Tris-(triphenylphosphin)-rhodium(I)-chlorid verwendet werden.

Die stereoselektive Anlagerung von Allylverbindungen der allgemeinen Formel III an 4,6-ungesättigte 3-Ketosteroide wird in absolutem Medium bei tiefen Temperaturen bis zu –85°C in organischen Lösungsmitteln, bevorzugt $CH_2Cl_2$, durchgeführt.

Das Alkenylierungsreagenz der Formel III wird ebenso wie der Lewissäurekatalysator im Vergleich zum 4,6-ungesättigten 3-Ketosteroid der Formel II im Überschuß eingesetzt.

Die selektive Hydrierung erfolgt nach an sich bekannten Methoden.

Die nachstehenden Beispiele sollen die Erfindung erläutern, ohne sie jedoch zu begrenzen.

## Beispiel 1

17β-Hydroxy-3-oxo-7α-propyl-17α-pregn-4-en-21-carbonsäure-Kaliumsalz

a) Eine Lösung von 5,0 g 3-Oxo-17α-pregn-4,6-dien-21-carbolacton in 350 ml absolutem Dichlormethan wird bei –70°C mit 7,7 ml Titantetrachlorid versetzt. Nach 5 Minuten wird eine Lösung von 10 g Allyltrimethylsilan in 20 ml Dichlormethan innerhalb 30 Minuten zugegeben und dann eine Stunde bei –70°C und 30 Minuten bei –20°C gerührt. Danach wird die Reaktionslösung mit 300 ml Wasser versetzt, mit Wasser neutralgewaschen, getrocknet und eingeengt. Das Rohprodukt wird durch Chromatographie an Kieselgel gereinigt. Man erhält 4,11 g 3-Oxo-7α-(2-propenyl)-17α-pregn-4-en-21, 17-carbolacton vom Schmelzpunkt 193-195°C.

b) Eine Lösung von 4,0 g 3-Oxo-7α-(2-propenyl)-17α-pregn-4-en-21, 17-carbolacton in 40 ml Tetrahydrofuran und 40 ml Methanol wird nach Zugabe von 400 mg Tris-(triphenylphosphin)-rhodium(I)-chlorid unter Normaldruck hydriert. Nach dem Verdampfen des Lösungsmittels wird der Rückstand an Kieselgel chromatographiert. Ausbeute : 2,98 g 3-Oxo-7α-propyl-17α-pregn-4-en-21, 17-carbolacton vom Schmelzpunkt 202-204°C.

c) Eine Lösung von 1,9 g 3-Oxo-7α-propyl-17α-pregn-4-en-21, 17-carbolacton in 25 ml 2-Propanol versetzt man mit 5 ml einer methanolischen 1 N Kaliumhydroxidlösung und erhitzt 30 Minuten unter Rückfluß. Nach dem Abkühlen läßt man die Reaktionslösung in 250 ml Diethylether einfließen. Das ausgefällte Produkt wird abfiltriert, mit Diethylether gewaschen und im Vakuum getrocknet.

Man erhält 1,57 g 17β-Hydroxy-3-oxo-7α-propyl-17α-pregn-4-en-21-carbonsäure-Kaliumsalz.

## Beispiel 2

17β-Hydroxy-7α-propyl-4-androsten-3-on

a) 5,8 g 17β-tert.-Butyldimethylsilyloxy-4,6-androstadien-3-on werden, wie im Beispiel 1 unter (a)

beschrieben, mit Allyltrimethylsilan umgesetzt. Das so erhaltene Rohprodukt wird in 100 ml Aceton gelöst, die Lösung wird mit 20 ml 20%iger Schwefelsäure versetzt, eine Stunde bei Raumtemperatur gerührt und in Eiswasser gegossen. Das ausgefällte Produkt wird isoliert und an Kieselgel chromatographiert. Man erhält 4,05 g 17β-Hydroxy-7α-(2-propenyl)-4-androsten-4-on vom Schmelzpunkt 220-222°C.

b) 3,95 g 17β-Hydroxy-7α-(2-propenyl)-4-androsten-3-on-werden, analog den im Beispiel 1 unter b) angegebenen Bedingungen, hydriert. Man erhält 3,15 g 17β-Hydroxy-7α-propyl-4-androsten-3-on mit einem Schmelzpunkt von 202-203°C.

## Beispiel 3

15β, 16β-Methylen-3-oxo-7α-propyl-17α-pregn-4-en-21,17-carbolacton

a) 5,0 g 15β, 16β-Methylen-3-oxo-17α-pregna-4,6-dien-21,17-carbolacton werden, entsprechend den im Beispiel 1 unter a) beschriebenen Bedingungen, in 15β, 16β-Methylen-3-oxo-7α-(2-propenyl)-17α-pregn-4-en-21, 17-carbolacton überführt. Ausbeute : 3,75 g.

b) 3,6 g 15β, 16β-Methylen-3-oxo-7α-(2-propenyl)-17α-pregn-4-en-21, 17-carbolacton werden, entsprechend den im Beispiel 1 unter b) beschriebenen Bedingungen, hydriert. Man erhält 2,92 g 15β, 16β-Methylen-3-oxo-7α-propyl-17α-pregn-4-en-21, 17-carbolacton. Schmelzpunkt 221-222°C.

## Beispiel 4

17β-Hydroxy-7α-propyl-4-androsten-3-on

Eine Lösung von 1,2 g 17β-tert.-Butyldimethylsilyloxy-4,6-androstadien-3-on in 70 ml absolutem Dichlormethan wird bei −70°C mit 1,5 ml Titantetrachlorid versetzt. Nach 5 Minuten wird eine Lösung von 5,0 g Allyltributylstannan hinzugegeben. Die Reaktionsmischung wird eine Stunde bei −70°C und 30 Minuten bei −20°C gerührt und anschließend mit 60 ml Wasser versetzt und mit Wasser neutralgewaschen. Das nach dem Trocknen und Eindampfen der organischen Phase erhaltene Produkt wird in 50 ml Aceton gelöst, die Lösung mit 10 ml 1 N Schwefelsäure versetzt und eine Stunde bei Raumtemperatur gerührt. Anschließend wird mit Ethylacetat verdünnt und mit Wasser gewaschen. Man trocknet, dampft ein, chromatographiert den Rückstand an Kieselgel und erhält 585 mg 17β-Hydroxy-7α-(2-propenyl)-4-androsten-3-on. Das so erhaltene Produkt läßt sich, wie im Beispiel 2 unter b) beschrieben, in 17β-Hydroxy-7α-propyl-4-androsten-3-on überführen.

## Beispiel 5

3-Oxo-7α-propyl-17α-pregn-4-en-21,17-carbolacton

a) Eine Lösung von 10,0 g 17β-Hydroxy-7α-propyl-4-androsten-3-on in 300 ml Dichlormethan wird bei Raumtemperatur zu einer gerührten Suspension von 50 g Pyridiniumchromat in 800 ml Dichlormethan getropft. Das Reaktionsgemisch wird weitere 2 Stunden bei Raumtemperatur gerührt, danach wird vom Ungelösten abfiltriert und das Filtrat mit Wasser gewaschen. Nach dem Trocknen und Eindampfen erhält man 8,2 g 7α-Propyl-4-androsten-3,17-dion.

b) Eine Lösung von 8,0 g 7α-Propyl-4-androsten-3, 17-dion in 100 ml 1-Methylpyrrolidon wird unter Eiskühlung mit 16 g Kaliummethylat versetzt und 5 Minuten gerührt. Man tropft eine Lösung von 2 ml Propargylalkohol in 5 ml 1-Methylpyrrolidon hinzu und rührt eine Stunde unter Eiskühlung. Anschließend bringt man das Reaktionsgemisch durch Zugabe von halbkonz. $H_2SO_4$ auf pH = 1, rührt eine Stunde bei Raumtemperatur und gießt in 1 Liter Wasser. Der entstehende Niederschlag wird abfiltriert, mit Wasser gewaschen und im Vakuum getrocknet. Ausbeute : 7,6 g 17β-Hydroxy-17α-(3-hydroxy-1-propinyl)-7α-propyl-4-androsten-3-on.

c) 7,5 g 17β-Hydroxy-17α-(3-hydroxy-1-propinyl)-7α-propyl-4-androsten-3-on löst man in 150 ml Tetrahydrofuran, versetzt mit 750 mg Tris-(triphenylphosphin)-rhodium(I)-chlorid und hydriert bei Normaldruck. Nach beendeter Wasserstoffaufnahme wird die Lösung im Vakuum eingeengt und der Rückstand in 300 ml Dimethylformamid gelöst. Zu dieser Lösung gibt man unter Rühren portionsweise 22,5 g Pyridiniumdichromat und rührt 4 Stunden bei Raumtemperatur. Anschließend verdünnt man mit 500 ml Ethylacetat, wäscht mit Wasser und engt im Vakuum ein. Der Rückstand, an Kieselgel chromatographiert, ergibt 6,1 g 3-Oxo-7α-propyl-17α-pregn-4-en-21, 17-carbolacton. Schmelzpunkt : 203-204°C.

Beispiel 6

17β-Hydroxy-7α-propyl-4-estren-3-on

Eine Lösung von 270 mg 17β-tert. -Butyldimethylsilyloxy-4,6-estradien-3-on in 21 ml absolutem Dichlormethan wird unter Argon auf −30°C gekühlt und mit 800 mg Aluminiumtrichlorid versetzt. Anschließend wird eine Lösung von 2.25 g Allyltributylstannan in 3 ml Dichlormethan innerhalb von 15 Minuten zugetropft. Die Reaktionsmischung wird 6.5 Stunden bei −30°C gerührt und danach mit 10 ml Wasser und 100 ml Diethylether versetzt. Die organische Phase wird abgetrennt, mit Wasser gewaschen, getrocknet und eingedampft. Der Rückstand wird in 10 ml Aceton gelöst, die Lösung mit 2 ml 1 N Schwefelsäure versetzt und eine Stunde bei Raumtemperatur gerührt. Anschließend wird mit Ethylacetat verdünnt, mit Wasser neutralgewaschen, getrocknet und eingedampft. Der Rückstand wird an Kieselgel chromatographiert. Man erhält 76 mg 17β-Hydroxy-7α-(2-propenyl)-4-estren-3-on. Das so erhaltene Produkt läßt sich, wie im Beispiel 2 unter b)beschrieben, in 17β-Hydroxy-7α-propyl-4-estren-3-on überführen.

## Ansprüche

1. Verfahren zur Herstellung von 7α-Propylsteroiden der allgemeinen Formel I,

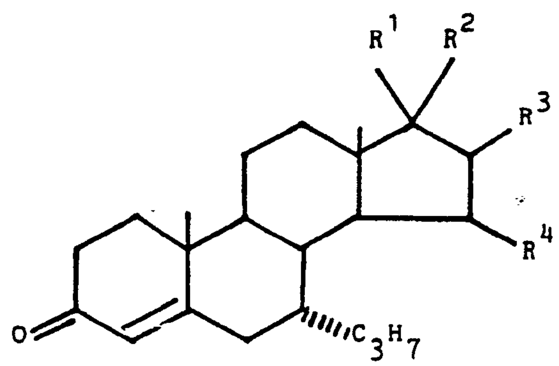

(I) ,

worin

$R^1$ eine β-Hydroxygruppe und

$R^2$ Wasserstoff oder den α-gebundenen Rest $-CH_2-CH_2COOR^5$,

$R^1$ und $R^2$ gemeinsam den Rest

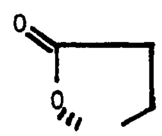

,

$R^3$, $R^4$ Wasserstoff,

$R^3$ und $R^4$ gemeinsam einen Methylenrest, der α- oder β-gebunden sein kann, und

$R^5$ Wasserstoff, Natrium oder Kalium bedeuten kann, dadurch gekennzeichnet, daß man 4,6-ungesättigte Steroidketone der allgemeinen Formel II

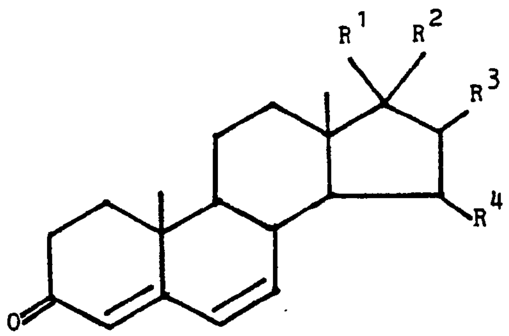

(II).

worin $R^1$-$R^4$ die oben genannten Bedeutungen haben, mit einer Allyltrialkylverbindung der allgemeinen Formel III,

$$CH_2=CH-CH_2-X(Alkyl)_3 \qquad (III).$$

worin X die Bedeutung Si oder Sn und Alkyl die

Bedeutung $C_{1-4}$-Alkyl haben, in Gegenwart einer Lewissäure umsetzt und anschließend die gebildete 7α-Propenyl-Seitenkette selektiv hydriert.

2. Verfahren nach Anspruch 1, dadurch gekennzeichnet, daß man als Lewissäuren $AlCl_3$, $SnCl_4$, $SnCl_2$, $BF_3$. Etherat, $AlCl_2(C_2H_5)_1$, $TiCl_4$ oder $AlCl(C_2H_5)_2$ verwendet.

3. Verfahren nach Anspruch 1, dadurch gekennzeichnet, daß man als Lewissäuren $TiCl_4$ oder $AlCl(C_2H_5)_2$ verwendet.

4. Verfahren nach Anspruch 1, dadurch gekennzeichnet, daß man für die selektive Hydrierung Tris-(triphenylphosphin)-rhodium(I)-chlorid verwendet.

5. Verfahren zur Herstellung von 17β-Hydroxy-7α-propyl-4-estren-3-on, dadurch gekennzeichnet, daß man 17β-tert.-Butyl-(dimethylsilyloxy-4,6-estradien-3-on mit Allyltributylstannan in Gegenwart von Aluminiumtrichlorid umsetzt und anschließend die gebildete 7α-(2-Propenyl)-Seitenkette selektiv hydriert.

**Claims**

1. Process for the preparation of 7α-propyl steroids of the general formula I

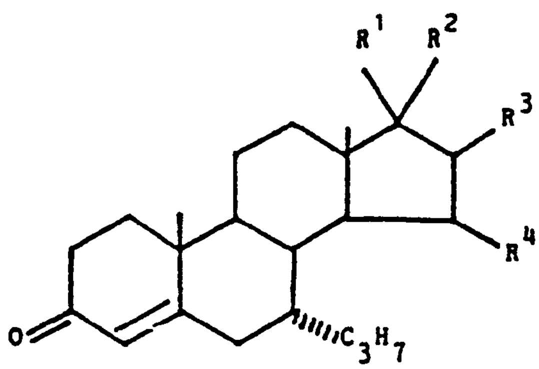

(I)

in which

$R^1$ may represent a β-hydroxy group and

$R^2$ may represent hydrogen or the α-bonded radical $-CH_2-CH_2COOR^5$,

$R^1$ and $R^2$ together may represent the radical

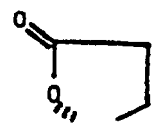

$R^3$ and $R^4$ may represent hydrogen,
$R^3$ and $R^4$ together may represent a methylene radical which may be α- or β-bonded, and
$R^5$ may represent hydrogen, sodium or potassium, characterised in that 4,6-unsaturated steroid ketones of the general formula II

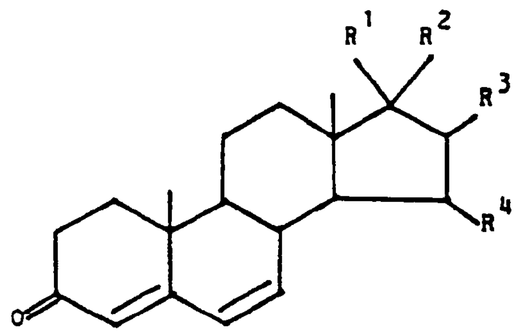

(II),

in which $R^1$-$R^4$ have the meanings mentioned above, are reacted with an allyltrialkyl compound of the general formula III

$$CH_2=CH-CH_2-X(alkyl)_3 \qquad (III),$$

in which
X represents Si or Sn and alkyl represents $C_{1-4}$-alkyl, in the presence of a Lewis acid and then the resulting 7α-propenyl side chain is selectively hydrogenated.

2. Process according to claim 1, characterised in that $AlCl_3$, $SnCl_4$, $SnCl_2$, $BF_3$ etherate, $AlCl_2(C_2H_5)_1$, $TiCl_4$ or $AlCl(C_2H_5)_2$ is used as Lewis acid.

3. Process according to claim 1, characterised in that $TiCl_4$ or $AlCl(C_2H_5)_2$ is used as Lewis acid.

4. Process according to claim 1, characterised in that tris-(triphenylphosphine)-rhodium(I) chloride is used for the selective hydrogenation.

5. Process for the preparation of 17β-hydroxy-7α-propyl-4-oestren-3-one, characterised in that 17β-tert.-butyl-dimethylsilyloxy-4,6-oestradien-3-one is reacted with allyltributylstannane in the presence of aluminium trichloride and then the resulting 7α-(2-propenyl) side chain is selectively hydrogenated.

**Revendications**

1. Procédé pour préparer des propyl-7α stéroïdes répondant à la formule générale I :

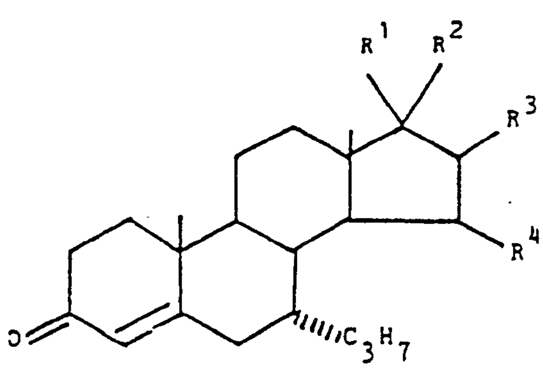

(I)

dans laquelle :
$R^1$ représente un radical β-hydroxy,
$R^2$ représente l'hydrogène ou un radical $-CH_2-CH_2-COOR^5$ lié avec la configuration α, ou
$R^1$ et $R^2$ forment ensemble un radical

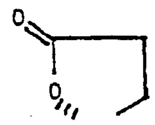

$R^3$ et $R^4$ représentent chacun l'hydrogène, ou
$R^3$ et $R^4$ représentent ensemble un radical méthylène qui peut être lié avec la configuration α ou la configuation β, et
$R^5$ représente l'hydrogène, le sodium ou le potassium, procédé caractérisé en ce qu'on fait réagir des oxostéroïdes insaturés en 4,6 qui répondent à la formule générale II :

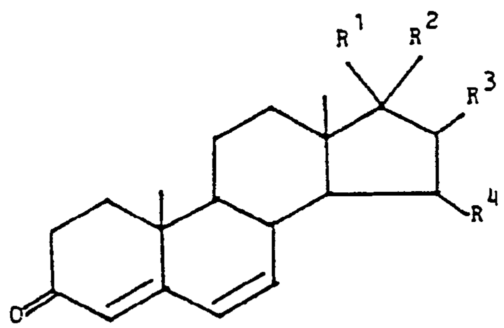

(II).

dans laquelle $R^1$ à $R^4$ ont les significations qui leur ont été données ci-dessus, avec un composé allyl-trialkylique répondant à la formule générale III :

$$CH_2 = CH - CH_2 - X(Alkyl)_3 \quad (III)$$

dans laquelle :
X représente Si ou Sn et Alkyl représente un radical alkyle contenant de 1 à 4 atomes de carbone, en présence d'un acide de Lewis, puis on hydrogène sélectivement la chaîne latérale propényle-7α qui s'est formée.

2. Procédé selon la revendication 1 caractérisé en ce qu'on utilise, comme acide de Lewis, $AlCl_3$, $SnCl_4$, $SnCl_2$, $BF_3$, le trifluorure de bore-oxyde de diéthyle, $AlCl_2(C_2H_5)$, $TiCl_4$ ou $AlCl_2(C_2H_5)$.

3. Procédé selon la revendication 1 caractérisé en ce qu'on utilise, comme acide de Lewis, $TiCl_4$ ou $AlCl(C_2H_5)_2$.

4. Procédé selon la revendication 1 caractérisé en ce qu'on utilise, pour l'hydrogénation sélective, le chlorure de tris-(triphénylphosphine)-rhodium(I).

5. Procédé pour préparer l'hydroxy-17β propyl-7α estrène-4 one-3, procédé caractérisé en ce qu'on fait réagir la tert-butyl-diméthylsilyloxy-17β estradiène-4,6 one-3 avec l'allyl-tributylstannane en présence de trichlorure d'aluminium, puis on hydrogène sélectivement la chaîne latérale (propène-2 yle)-7α formée.